# EUROPÄISCHE PATENTSCHRIFT

(11) **EP 3 875 953 B1**
(45) Veröffentlichungstag und Bekanntmachung des Hinweises auf die Patenterteilung: **12.07.2023**
(21) Anmeldenummer: 20161355.1
(22) Anmeldetag: 06.03.2020
(51) Int. Cl.: G01N 33/02, G01N 17/00

(54) **BESCHLEUNIGTER LAGERTEST**
ACCELERATED STORAGE TEST
TEST DE STOCKAGE ACCÉLÉRÉ

(43) Veröffentlichungstag der Anmeldung: 08.09.2021
(73) Patentinhaber: DMK Deutsches Milchkontor GmbH, 27404 Zeven (DE)
(72) Erfinder: Bode, Katja, 21279 Hollenstedt (DE); Zink, Ralf, 26160 Bad Zwischenahn (DE)
(74) Vertreter: Fabry, Bernd

(56) Entgegenhaltungen:
- DE-A1-102014 112 953
- JP-A- H0 391 468
- JP-A- 2014 077 555
- PETERSEN M A ET AL: "Comparison of normal and accelerated storage of commercial orange juice - changes in flavour and content of volatile compounds", FOOD QUALITY AND PREFERENCE, ELSEVIER, AMSTERDAM, NL, Bd. 9, Nr. 1/2, 25. Februar 1998 (1998-02-25), Seite 43, XP002378268, ISSN: 0950-3293, DOI: 10.1016/S0950-3293(97)00027-X

## Beschreibung

### GEBIET DER ERFINDUNG

Die Erfindung befindet sich auf dem Gebiet der Lebensmitteltechnologie und betrifft ein Verfahren zur beschleunigten Bestimmung der Qualität und insbesondere der Haltbarkeit von Lebensmitteln sowie eine entsprechende Vorrichtung.

### TECHNOLOGISCHER HINTERGRUND

Eine der größten Anforderungen in der Lebensmittelindustrie besteht darin, ihre Produkte nicht nur qualitativ einwandfrei herzustellen und in Verkehr zu bringen, sondern diese Qualität auch für einen ausreichend langen Zeitraum sicherzustellen. Neben der mikrobiologischen Haltbarkeit, die durch geeignete Verfahren, wie Erwärmen, aseptisches Abfüllen und Verpackung, sowie Konservieren gewährleistet werden kann, müssen insbesondere auch chemische oder physikalische Veränderungen des Füllgutes während der Lagerzeit vermieden werden, da diese im günstigsten Fall nur zu Aromaveränderungen führen können, im ungünstigsten Fall jedoch die Nahrungsmittel verderben. Einer der wichtigsten Faktoren, die die Qualität von Lebensmittel beeinflussen, stellt die Permeation von Stoffen, wie insbesondere Sauerstoff, durch das Packmittel dar, die infolge von Oxidation Farbe, Geschmack des Lebensmittels oder beides beeinträchtigen kann. Die verwendeten Verpackungen müssen daher einen optimalen Produktschutz gewähren und sowohl im Hinblick auf die Materialauswahl als auch das Verpackungsdesign hohen packgutspezifischen Anforderungen genügen. Insbesondere muss innerhalb der deklarierten Mindesthaltbarkeit eine einwandfreie Qualität des Produkts sichergestellt sein.

Zur Ermittlung der Mindesthaltbarkeit stehen neben Materialprüfungstests und theoretischen Berechnungsmodellen auch Standardqualifikationstests, wie etwa Produktlagertests in Echtzeit zur Verfügung. Dabei werden die zu untersuchenden Packmittel befüllt und über den Zeitraum der dafür vorgesehenen Mindesthaltbarkeit unter kontrollierten Bedingungen gelagert. Dies bedeutet allerdings, dass eine hinreichend gesicherte Aussage über die Tauglichkeit eines Verpackungsdesigns für ein bestehendes Produkt oder im Umkehrschluss einer Produktneuentwicklung für eine bestehende Verpackungsart erst nach Ablauf der vorgesehenen Mindesthaltbarkeit getroffen werden kann. In dieser sehr langen Testzeit von mehreren Monaten zeigt sich der wesentliche Nachteil dieser Testmethode. Bedingt durch die immer größer werdende Produktvielfalt, immer kürzer werdenden Produktzyklen bei gleichzeitig hoher Erwartung des Verbrauchers bezüglich Produktqualität und -sicherheit besteht im Wettbewerb der Anbieter von Produkten die Notwendigkeit zur Reduzierung der benötigten Entwicklungszeit.

### RELEVANTER STAND DER TECHNIK

Aus dem Stand der Technik sind bereits einschlägige Verfahren bekannt, mit deren Hilfe man die Haltbarkeit von Lebensmitteln in einer Verpackung bestimmen kann. So wird beispielsweise in der EP 1626275 B1 (WILD) ein Verfahren zur Bewertung der Produkthaltbarkeit in einem Packmittel vorgeschlagen, das die folgenden Schritte umfasst:
a) Einbringen eines mit Füllgut gefüllten, verschlossenen Packmittels (4) in eine Überdruckkammer (1),
b) Lagern des Packmittels (4) in der Überdruckkammer (1) über einen Zeitraum t₁ bei Überdruck p₁ und einer bestimmten Temperatur T₁ in einer Testgasatmosphäre, wodurch eine definierte Menge Q an Testgas in das Packmittel permeiert,
c) Lagern des Packmittels (4) über einen bestimmten Zeitraum t₂ unter Wärme und/oder Lichteinfluss, und
d) analytische und/oder sensorische Untersuchung des Füllguts.

Nachteilig bei dem Verfahren ist der hohe Apparateaufwand, zudem lässt das Funktionsprinzip - Nachbildung der in das Füllgut eingebrachten Sauerstoffmenge und Beschleunigung der Reaktion durch erhöhten Druck- letztlich nur eine grobe Schätzung zu, die für eine qualitative Aussage vielfach ungeeignet ist, zumal bekannt ist, dass für viele Polymer/Gas-Systeme Löslichkeits- und Diffusionskoeffizient der permeirenden Substanz im Polymer (z.B. einer Plastikflasche) von Konzentration und Druck abhängig werden, wenn die Untersuchung unterhalb der Glastemperatur Tg der Polymere und/oder der kritischen Temperatur des Gases durchgeführt werden [MÜLLER, K. "Sauerstoff-Durchlässigkeit von Kunststoffflaschen und Verschlüssen" Dissertation TU München (2003)]

Eine völlig andere Methode, die Haltbarkeit eines verpackten Lebensmittels für den Verbraucher optisch anzuzeigen, ist Gegenstand der Schutzrechte EP 2378354 B1**,** EP 2581785 B1 sowie WO 2009 056591 A1 (UNI MÜNSTER). Diese betreffen eine Sensorvorrichtung, konkret einen elektrochemischen Prozessor, der mit dem Öffnen der Verpackung kurzgeschlossen wird. Es bildet sich eine Aluminiumoxidschicht, deren Fortschreiten proportional zur Haltbarkeit des Produktes verläuft. Eine Temperaturerhöhung führt beispielsweise dazu, dass die Oxidbildung beschleunigt wird und spiegelt damit natürlich auch den abnehmenden Frischegrad des Produktes wieder. Die Sensoren sind unter der Bezeichnung "Open Monitor" von Global Innovations Deutschland GmbH erhältlich. Diese Lösung macht es indes erforderlich, jede einzelne Verpackung mit einem separaten Sensor auszustatten, was ebenfalls technisch aufwendig ist.

Eine weitere Methode und Vorrichtung sind aus DE 10 2014 112953 A1 bekannt.

### AUFGBE DER ERFINDUNG

Eine erste Aufgabe der vorliegenden Erfindung hat daher darin bestanden, bereits aus dem Stand der Technik bekannte beschleunigte Lagertests dahingehend zu verbessern, dass Ergebnisse mit höherer Aussagefähigkeit erhalten werden. Dies trifft insbesondere für Lebensmittel zu, die bereits verpackt vorliegen.

Eine zweite Aufgabe ist darin zu sehen, eine Vorrichtung zu entwerfen, mit deren Hilfe ein solcher verbesserter Lagertest durchgeführt werden kann.

### BESCHREIBUNG DER ERFINDUNG

Ein erster Gegenstand der Erfindung betrifft ein Verfahren zur beschleunigten Bestimmung der Qualität von Lebensmitteln umfassend oder bestehend aus den folgenden Schritten:
(i) Bereitstellen einer ersten Lebensmittelprobe;
(ii) Lagerung der ersten Lebensmittelprobe bei der Temperatur T1 über die Zeitdauer t1;
(iii) Sensorische und/oder analytische Beurteilung der ersten Lebensmittelprobe durch regelmäßige Probennahme unter Erhalt einer Kalibrierkurve;
(iv) Bereitstellen einer gleichartigen zweiten Lebensmittelprobe;
(v) Lagerung der zweiten Lebensmittelprobe bei der Temperatur T2 über die Zeitdauer t2;
(vi) Sensorische und/oder analytische Beurteilung der zweiten Lebensmittelprobe nach Ablauf der Zeitdauer t2 ; und
(vii) Bestimmung der Qualität durch Vergleich der sensorischen und/oder analytischen Beurteilung der zweiten Lebensmittelprobe nach Ablauf der Zeitdauer t2 mit der Kalibrierkurve,
wobei
(a) die Temperatur T2 um mindestens 5 °C höher als die Temperatur T1 liegt;
(b) die Zeitdauer t1 mindestens 5mal länger als die Zeitdauer t2 ist;
(c) die zweite Lebensmittelprobe bei einem Druck von etwa 0,1 bis etwa 20 bar gelagert und
(d) während der Lagerung einer Rotations- oder Pendelbewegung ausgesetzt wird.

Überraschenderweise wurde gefunden, dass die Ergebnisse aus beschleunigten Lagertest nach dem Stand der Technik deutlich genauer und damit aussagefähiger werden, wenn man die Lagerung des Testproduktes zum einen unter Druck durchführt und die Probe, vorzugsweise in verpacktem Zustand, über die gesamte Lagerzeit ständig bewegt, vorzugsweise einer Rotations- oder Pendelbewegung aussetzt. Auf diese Weise lässt sich zudem die Testdauer signifikant verkürzen, da belastbare Ergebnisse in deutlich kürzeren Zeiten erzielt werden.

### LEBENSMITTEL

Das erfindungsgemäße Verfahren ist grundsätzlich für alle Arten von Lebensmitteln und insbesondere auch verpackten Lebensmitteln geeignet.

Konkrete Beispiele umfassen:
- **Milchprodukte,** wie beispielsweise, Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolysierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke;
- **Käseprodukte,** wie beispielsweise Hartkäse, Weichkäse oder pasta filata-Typen (z.B. Mozzarella)
- **Fleischprodukte,** wie beispielsweise Schinken, Frischwurst- oder Rohwurstzubereitungen, gewürzte oder marinierte Frisch- oder Pökelfleischprodukte sowie
- **Getränke,** wie beispielsweise Kaffee, Tee, Eistee, Wein, weinhaltige Getränke, Bier, bierhaltige Getränke, Liköre, Schnäpse, Weinbrände, (karbonisierte) fruchthaltige Limonaden, (karbonisierte) isotonische Getränke und mit Geschmacksstoffen angereichertes Mineralwasser, (karbonisierte) Erfrischungsgetränke, Nektare, Schorlen, Obst- und Gemüsesäfte, Frucht- oder Gemüsesaftzubereitungen, Instantgetränke, wie beispielsweise Instant-Kakao-Getränke, Instant-Tee-Getränke, Instant-Kaffeegetränke oder Instant-Fruchtgetränke.

Besonders vorteilhaft ist das Verfahren zur Untersuchung von Lebensmitteln, die schon herstellungsbedingt für eine längere Lagerung von beispielsweise 9 bis 12 Monaten vorgesehen sind, weil sich auf diese Weise der Beurteilungsspielraum auf 1 bis 4 Wochen verkürzen lässt. Typische Produkte sind hocherhitzte Milch ("UHT-Milch"), hocherhitzter Joghurt oder Milchpulver. Gleiches trifft für Instantprodukte in Summe zu.

### DURCHFÜHRUNG DES VERFAHRENS

Wie eingangs bereits erläutert, simuliert der beschleunigte Lagertest die Alterung eines Produktes. Am Ende der Alterungslagerung wird die Qualität des Produktes sensorisch und/oder analytisch beurteilt und mit Hilfe einer Kalibrierkurve einem Muster zugeordnet, das diesen Qualitätszustand ohne Beschleunigung, d.h. unter üblichen Lagerbedingungen erreicht hätte. Dem Testprodukt, das beschleunigt gealtert wurde, wird also sein "echtes" Alter zugeordnet.

Die sensorische Beurteilung ist dabei subjektiv und wird vorzugsweise nach der so genannten "Degree of Difference"-Methode ("DoD") durch ein geschultes Panel von Prüfern durchgeführt. Die Veränderung wird relativ zu der durchschnittlichen Qualität der Ausgangsprobe ermittelt und umfasst zum einen das gesamte Erscheinungsbild (Farbe, Geruch, Sedimentation, Entmischung und dergleichen) sowie zum anderen die geschmackliche Qualität.

Die weitere Beurteilung der Produktqualität erfolgt sowohl mittels physikalischer als auch chemischer Analysen wie Bestimmung von pH-Wert, Partikelgröße, Farbe, Restsauerstoffgehalt, Oberflächenspannung, Sedimentations- und Aufrahmgeschwindigkeit, Proteinabbauprodukte und dergleichen.

Im ersten Schritt wird eine Kalibrierkurve erstellt. Hierzu wird eine erste Lebensmittelprobe einer für sie typischen Lagerung unterworfen, d.h. bei Temperaturen von etwa - 30 bis etwa 100 °C, vorzugsweise etwa 4 bis etwa 45 °C und insbesondere etwa 8 bis etwa 37 °C. Die typische Lagerzeit kann einen Zeitraum von etwa 1 bis etwa 24 Monaten umfassen, abhängig davon, welches Mindesthaltbarkeitsdatum herstellungsbedingt angestrebt wird. Bei Frischeprodukten liegt dies naturgemäß eher im Bereich von 1 Monat, gerade bei hocherhitzten und insbesondere getrockneten Produkten eher im Bereich von 6 bis 24 und vorzugsweise 8 bis 12 Monaten. Auch Produkte mit kürzerer Haltbarkeit als 1 Monat können auf diese Weise getestet werden, jedoch lohnt sich der Aufwand in der Regel nicht.

Während der Lagerung werden in regelmäßigen Abständen Proben entnommen und wie oben beschrieben sensorisch und/oder analytisch beurteilt. So wird eine Kalibrierkurve erhalten, die die Qualitätsverschlechterung über die Zeit angibt. Auf diese Weise lässt sich auch eine Mindesthaltbarkeit angeben; dabei handelt es sich um den Zeitpunkt, ab dem die Qualität der Probe gegenüber der Ausgangsqualität einen vorgegeben Wert unterschreitet.

Im zweiten Schritt erfolgt die Lagerung unter beschleunigten Bedingungen. Die zweite Lebensmittelprobe ist dabei gleichartig zur ersten, d.h. in Beschaffenheit, Alter, Menge und Verpackung identisch. Sie wird bei deutlich höheren Temperaturen nämlich bei etwa 30 bis 120 °C gelagert. Erfolgt die Lagerung der Referenzprobe beispielsweise im Bereich von 4 bis 20 °C, dann empfiehlt es sich die Testprobe bei etwa 25 bis etwa 60 °C und vorzugsweise bei etwa 30 °C zu lagern.

Sinn des Verfahrens ist es, die Lagerzeit um mindestens den Faktor 5 zu beschleunigen. Typisch sind daher Lagerzeiten für die zweite Lebensmittelprobe von etwa 1 bis etwa 28 Tagen.

Es hat sich ferner als vorteilhaft für die Aussagekraft und Genauigkeit der Testwerte erwiesen, wenn man die Lagerung der zweiten Lebensmittelprobe druckabhängig durchführt, beispielsweise bei einem Unterdruck von etwa 0,1 bis etwa 0,9 bar oder einem Überdruck von etwa 1,1 bis etwa 20 bar und vorzugsweise bei etwa 2 bis etwa 10 bar.

Eine weitere bevorzugte Ausführungsform besteht darin, die Probe während der Lagerung mit Feuchtigkeit, beispielsweise etwa 1 bis 5 Gew.-% bezogen auf das Probengewicht zu beaufschlagen und/oder die Lagerung unter Begasung, beispielsweise mit Luft, Sauerstoff oder Stickstoff durchzuführen.

Vorzugsweise wird die Lagerung daher in einer temperierbaren Druckkammer durchgeführt, die man in eine Rotations- oder Pendelbewegung versetzen kann. Alternativ können die Proben auch auf einer beweglichen Platte gelagert werden, die in eine Rüttelbewegung versetzt wird.

### VORRICHTUNG

Ein weiterer Gegenstand der Erfindung betrifft eine Vorrichtung zur Durchführung des oben beschriebenen erfindungsgemäßen Verfahrens, umfassend oder bestehend aus:
(i) einer temperierbaren Druckkammer,
(ii) einem Einsatz zur Aufnahme der Proben,
(iii) einem Motor, der entweder die Druckkammer oder den darin befindlichen Probeneinsatz in Pendelbewegung versetzen kann, sowie gegebenenfalls
(iv) einem Einlass für Beaufschlagung der Druckkammer mit Feuchtigkeit und/oder Gasen.

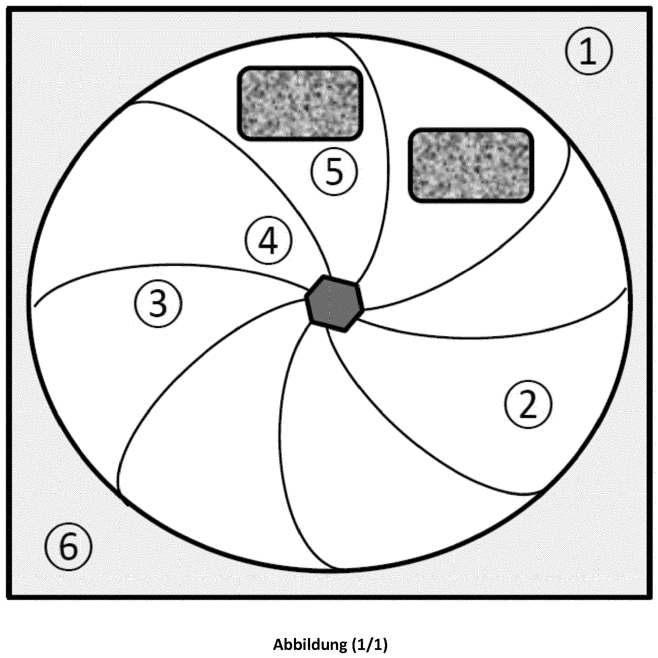

Die Vorrichtung wird näher in **Abbildung 1** illustriert. Vorzugsweise handelt es sich dabei um einen Pendelbewegungen ausführenden Kessel, der einen Korb zum Einsatz der Proben enthält, ähnlich einer Waschmaschine. Alternativ kann auch der Korb bewegt werden, während die Druckkammer still steht. In der Abbildung bedeuten:
- 1: Druckkammer
- 2: Korbeinsatz
- 3: Probenhalter
- 4: Motorwelle
- 5: Proben (z.B. 250 ml UHT-Milch-Packung)
- 6: Heizmantel

### BEISPIEL 1

### Erstellen der Kalibrierkurve

1 Liter UHT-Milch wurde bei 4 °C über einen Zeitraum von 12 Monaten in einer lichtundurchlässigen Kartonverpackung gelagert. Im Abstand von einem Monat wurden Proben entnommen und von einem Panel bestehend aus 5 erfahrenen Prüfern sensorisch auf einer Skala von (0) = keine Änderung bis (5) = sehr deutliche Änderung beurteilt. In **Tabelle 1** sind die Mittelwerte der Messungen angegeben. Aus diesen wurde jeweils ein weiterer Mittelwert über alle Parameter gebildet, dieser jeweils mit dem Startpunkt verglichen und als Abweichung ("DoD") angegeben.

**Tabelle 1**

| Kalibrierkurve mit DoD-Werten | | | | | | | | | | | | | |
|---|---|---|---|---|---|---|---|---|---|---|---|---|---|
| **Lagerzeit (m)** | **0** | **1** | **2** | **3** | **4** | **5** | **6** | **7** | **8** | **9** | **10** | **11** | **12** |
| Farbe | | 1 | 1 | 1 | 1 | 2 | 2 | 2 | 2 | 3 | 3 | 3 | 3 |
| Geruch | | 1 | 1 | 1 | 2 | 2 | 2 | 2 | 2 | 2 | 3 | 3 | 3 |
| Entmischung | | 1 | 1 | 1 | 1 | 1 | 1 | 1 | 1 | 1 | 1 | 1 | 1 |
| Geschmack | | 1 | 1 | 1 | 2 | 2 | 2 | 3 | 3 | 3 | 3 | 3 | 4 |
| Mittelwert | | 1 | 1 | 1 | 1,5 | 1,75 | 1,75 | 2 | 2 | 2,25 | 2,5 | 2,5 | 2,75 |

### BEISPIEL 2

### Beschleunigter Lagertest

Insgesamt 6 Proben UHT-Milch mit jeweils 250 ml wurde in einer temperierbaren Drucckammer gemäß Abbildung 1 platziert und bei 40 °C und 2 bar über einen Zeitraum von 1 Monat gelagert. Die Druckkammer rotierte dabei mit einer Geschwindigkeit von 10 UpM. Anschließend wurden die 6 Muster sensorisch wie oben erläutert beurteilt. Es ergab sich ein Mittelwert von 2,25. Die Lagerung von UHT-Milch unter den angegebenen Bedingungen führt also zu einem Produkt, das einer Standard-Lagerung von UHT-Milch bei 4 °C nach 9 Monaten entspricht.

## Patentansprüche

1. Verfahren zur beschleunigten Bestimmung der Qualität von Lebensmitteln umfassend oder bestehend aus den folgenden Schritten:
(i) Bereitstellen einer ersten Lebensmittelprobe;
(ii) Lagerung der ersten Lebensmittelprobe bei der Temperatur T1 über die Zeitdauer t1;
(iii) Sensorische und/oder analytische Beurteilung der ersten Lebensmittelprobe durch regelmäßige Probennahme unter Erhalt einer Kalibrierkurve;
(iv) Bereitstellen einer gleichartigen zweiten Lebensmittelprobe;
(v) Lagerung der zweiten Lebensmittelprobe bei der Temperatur T2 über die Zeitdauer t2;
(vi) Sensorische und/oder analytische Beurteilung der zweiten Lebensmittelprobe nach Ablauf der Zeitdauer t2; und
(vii) Bestimmung der Qualität durch Vergleich der sensorischen Qualität der zweiten Lebensmittelprobe nach Ablauf der Zeitdauer t2 mit der Kalibrierkurve,
wobei
(a) die Temperatur T2 um mindestens 5 °C höher als die Temperatur T1 liegt;
(b) die Zeitdauer t1 mindestens 5mal länger als die Zeitdauer t2 ist;
(c) die zweite Lebensmittelprobe bei einem Druck von etwa 0,1 bis 20 bar gelagert und
(d) während der Lagerung einer Rotations- oder Pendelbewegung ausgesetzt wird.

2. Verfahren nach Anspruch 1, **dadurch gekennzeichnet, dass** die Lebensmittel Milchprodukte, Käseprodukte, Fleischprodukte oder Getränke darstellen.

3. Verfahren nach den Ansprüchen 1 und/oder 2, **dadurch gekennzeichnet, dass** die Lebensmittel verpackt sind.

4. Verfahren nach mindestens einem der Ansprüche 1 bis 3, **dadurch gekennzeichnet, dass** man die erste Lebensmittelprobe bei Temperaturen von etwa 4 bis etwa 100 °C lagert.

5. Verfahren nach mindestens einem der Ansprüche 1 bis 4, **dadurch gekennzeichnet, dass** man die erste Lebensmittelprobe über einen Zeitraum von etwa 1 bis etwa 24 Monaten lagert.

6. Verfahren nach mindestens einem der Ansprüche 1 bis 5, **dadurch gekennzeichnet, dass** man die zweite Lebensmittelprobe bei Temperaturen von etwa 10 bis etwa 120 °C lagert.

7. Verfahren nach mindestens einem der Ansprüche 1 bis 6, **dadurch gekennzeichnet, dass** man die zweite Lebensmittelprobe über einen Zeitraum von etwa 1 bis etwa 28 Tage lagert.

8. Verfahren nach mindestens einem der Ansprüche 1 bis 7, **dadurch gekennzeichnet, dass** man, die zweite Lebensmittelprobe bei einem Druck von etwa 0,5 bis etwa 20 bar lagert.

9. Verfahren nach mindestens einem der Ansprüche 1 bis 8, **dadurch gekennzeichnet, dass** man die zweite Lebensmittelprobe während der Lagerung mit Feuchtigkeit beaufschlagt.

10. Verfahren nach mindestens einem der Ansprüche 1 bis 9, **dadurch gekennzeichnet, dass** man die zweite Lebensmittelprobe während der Lagerung mit Gas beaufschlagt.

11. Verfahren nach mindestens Anspruch 10, **dadurch gekennzeichnet, dass** man Luft, Sauerstoff oder Stickstoff einsetzt.

12. Verfahren nach mindestens einem der Ansprüche 1 bis 11, **dadurch gekennzeichnet, dass** man die zweite Lebensmittelprobe in einer temperierbaren Druckkammer rotiert oder einer Pendelbewegung aussetzt.

13. Verfahren nach mindestens einem der Ansprüche 1 bis 12, **dadurch gekennzeichnet, dass** die sensorische und/oder analytische Beurteilung das optische Erscheinungsbild, die Produktstabilität und den Geschmack der Lebensmittelproben umfasst.

14. Verfahren nach mindestens einem der Ansprüche 1 bis 13, **dadurch gekennzeichnet, dass** die sensorische und/oder analytische Beurteilung relativ zur ersten Lebensmittelprobe erfolgt.

15. Vorrichtung zur Durchführung des beschleunigten Lagertests nach mindestens einem der Ansprüche 1 bis 14, umfassend oder bestehend aus
(i) einem Einsatz zur Aufnahme der Proben,
(ii) einem Motor, der entweder die Druckkammer oder den darin befindlichen Probeneinsatz in Pendelbewegung versetzen kann, sowie gegebenenfalls
(iii) einem Einlass für Beaufschlagung der Druckkammer mit Feuchtigkeit und/oder Gasen, **dadurch gekennzeichnet, dass** die Vorrichtung weiterhin umfasst:
(iv) eine temperierte Druckkammer.

## Claims

1. A method for accelerated determination of food quality comprising or consisting of the following steps:
(i) providing a first food sample;
(ii) storing the first food sample at temperature T1 for time t1;
(iii) sensory and/or analytical evaluation of the first food sample by periodic sampling to obtain a calibration curve;
(iv) providing a similar second food sample;
(v) storing the second food sample at temperature T2 for time t2;
(vi) sensory and/or analytical evaluation of the second food sample at the end of the time period t2; and
(vii) determining the quality by comparing the sensory quality of the second food sample at the end of the time period t2 with the calibration curve,
wherein
(a) the temperature T2 is at least 5 °C higher than the temperature T1
(b) the time period t1 is at least 5 times longer than the time period t2;
(c) the second food sample is stored at a pressure of about 0.1 to 20 bar; and
(d) is subjected to a rotational or pendular movement during storage.

2. Method according to claim 1, **characterized in that** the foodstuffs are dairy products, cheese products, meat products or beverages.

3. Method according to claims 1 and/or 2, **characterised in that** the foodstuffs are packaged.

4. A method according to at least one of claims 1 to 3, **characterized in that** the first food sample is stored at temperatures from about 4 to about 100 °C.

5. A method according to at least one of claims 1 to 4, **characterised in that** the first food sample is stored for a period of about 1 to about 24 months.

6. A method according to at least one of claims 1 to 5, **characterized in that** the second food sample is stored at temperatures from about 10 to about 120 °C.

7. A method according to at least one of claims 1 to 6, **characterized in that** the second food sample is stored for a period of about 1 to about 28 days.

8. Method according to at least one of claims 1 to 7, **characterised in that** the second food sample is stored at a pressure of about 0.5 to about 20 bar.

9. Method according to at least one of claims 1 to 8, **characterised in that** moisture is applied to the second food sample during storage.

10. Method according to at least one of claims 1 to 9, **characterised in that** gas is applied to the second food sample during storage.

11. Process according to at least claim 10, **characterized in that** air, oxygen or nitrogen is used.

12. Method according to at least one of claims 1 to 11, **characterised in that** the second food sample is rotated in a temperature-controlled pressure chamber or subjected to a pendular movement.

13. Method according to at least one of claims 1 to 12, **characterised in that** the sensory and/or analytical assessment comprises the visual appearance, the product stability and the taste of the food samples.

14. Method according to at least one of claims 1 to 13, **characterised in that** the sensory and/or analytical evaluation is carried out relative to the first food sample.

15. A device for performing the accelerated storage test according to at least one of claims 1 to 14, comprising or consisting of
(i) an insert for receiving the samples,
(ii) a motor capable of oscillating either the pressure chamber or the sample insert therein, and optionally
(iii) an inlet for admission of moisture and/or gases to the pressure chamber, **characterised in that** the apparatus further comprises
(iv) a temperature-controlled pressure chamber.

## Revendications

1. Procédé de détermination accélérée de la qualité de produits alimentaires, comprenant ou consistant en les étapes suivantes :
(i) fourniture d'un premier échantillon de produit alimentaire ;
(ii) stockage du premier échantillon de produit alimentaire à la température T1 pendant la durée t1 ;
(iii) évaluation par capteurs et/ou analyses du premier échantillon de produit alimentaire par prélèvement régulier d'échantillons en vue d'obtenir une courbe d'étalonnage ;
(iv) fourniture d'un deuxième échantillon de produit alimentaire de même nature ;
(v) stockage du deuxième échantillon de produit alimentaire à la température T2 pendant la durée t2 ;
(vi) évaluation par capteurs et/ou analyses du deuxième échantillon de produit alimentaire après écoulement de la durée t2 ; et
(vii) détermination de la qualité par comparaison de la qualité obtenue par capteurs du deuxième échantillon de produit alimentaire après écoulement de la durée t2, à la courbe d'étalonnage,
dans lequel
(a) la température T2 est supérieure à la température T1 d'au moins 5 °C ;
(b) la durée t1 est au moins 5 fois plus longue que la durée t2 ;
(c) le deuxième échantillon de produit alimentaire est stocké à une pression d'environ 0,1 à 20 bars, et
(d) est soumis à un mouvement de rotation ou de pendule pendant le stockage.

2. Procédé selon la revendication 1, **caractérisé en ce que** les produits alimentaires sont des produits laitiers, des produits fromagers, des produits carnés ou des boissons.

3. Procédé selon les revendications 1 et/ou 2, **caractérisé en ce que** les produits alimentaires sont emballés.

4. Procédé selon au moins l'une des revendications 1 à 3, **caractérisé en ce que** l'on stocke le premier échantillon de produit alimentaire à des températures d'environ 4 à environ 100°C.

5. Procédé selon au moins l'une des revendications 1 à 4, **caractérisé en ce que** l'on stocke le premier échantillon de produit alimentaire pendant une durée d'environ 1 à environ 24 mois.

6. Procédé selon au moins l'une des revendications 1 à 5, **caractérisé en ce que** l'on stocke le deuxième échantillon de produit alimentaire à des températures d'environ 10 à environ 120°C.

7. Procédé selon au moins l'une des revendications 1 à 6, **caractérisé en ce que** l'on stocke le deuxième échantillon de produit alimentaire pendant une durée d'environ 1 à environ 28 jours.

8. Procédé selon au moins l'une des revendications 1 à 7, **caractérisé en ce que** l'on stocke le deuxième échantillon de produit alimentaire à une pression allant d'environ 0,5 à environ 20 bars.

9. Procédé selon au moins l'une des revendications 1 à 8, **caractérisé en ce que** l'on expose le deuxième échantillon de produit alimentaire à de l'humidité pendant le stockage.

10. Procédé selon au moins l'une des revendications 1 à 9, **caractérisé en ce que** l'on expose le deuxième échantillon de produit alimentaire à un gaz pendant le stockage.

11. Procédé selon au moins la revendication 10, **caractérisé en ce que** l'on utilise de l'air, de l'oxygène ou de l'azote.

12. Procédé selon au moins l'une des revendications 1 à 11, **caractérisé en ce que** l'on soumet à une rotation ou à un mouvement pendulaire le deuxième échantillon de produit alimentaire dans une chambre de pression pouvant être thermorégulée.

13. Procédé selon au moins l'une des revendications 1 à 12, **caractérisé en ce que** l'évaluation par capteurs et/ou analyses comprend l'aspect optique, la stabilité du produit et le goût des échantillons de produits alimentaires.

14. Procédé selon au moins l'une des revendications 1 à 13, **caractérisé en ce que** l'évaluation par capteurs et/ou analyses est effectuée relativement au premier échantillon de produit alimentaire.

15. Dispositif pour la mise en oeuvre du test de stockage accéléré selon au moins l'une des revendications 1 à 14, comprenant ou consistant en
(i) un élément d'insertion destiné à recevoir les échantillons,
(ii) un moteur capable de soumettre à un mouvement pendulaire soit la chambre de pression, soit l'élément d'insertion d'échantillon qui s'y trouve, et le cas échéant,
(iii) un orifice d'entrée permettant d'alimenter la chambre de pression en humidité et/ou en gaz,
**caractérisé en ce que** le dispositif comprend en outre:
(iv) une chambre de pression thermorégulée.
